# EUROPEAN PATENT SPECIFICATION

(11) **EP 2 714 713 B1**
(45) Date of publication and mention of the grant of the patent: **09.05.2018**
(21) Application number: 12790188.2
(22) Date of filing: 24.05.2012
(51) Int. Cl.: C07K 1/18, C07K 16/28, C07K 1/20, C07K 1/22, C07K 1/16, C07K 16/00, A61K 39/395

(54) **PURIFICATION OF ANTI-CD20 ANTIBODIES**
REINIGUNG VON ANTI-CD20 ANTIKÖRPERN
PURIFICATION D'ANTICORPS ANTI-CD20

(30) Priority: 26.05.2011 IN 1783CH2011
(43) Date of publication of application: 09.04.2014
(73) Proprietor: Dr. Reddy's Laboratories Limited, R R District 500072 (IN)
(72) Inventor: JAHAGIRDAR, Kishore, Miyapur Hyderabad 500050 (IN); GUPTA, Neeru, Ambala 134003 (IN)
(74) Representative: Bates, Philip Ian
(86) International application number: PCT/IB2012/052594
(87) International publication number: WO 2012/160530

(56) References cited:
- WO-A1-02/12455
- WO-A1-2009/058812
- WO-A1-2009/058812
- WO-A1-2011/009623
- WO-A1-2011/037983
- WO-A2-01/32878
- WO-A2-2004/024866
- GRAF H ET AL: "Ion exchange resins for the purification of monoclonal antibodies from animal cell culture", BIOSEPARATION, KLUWER ACADEMIC PUBLISHERS, DORDRECHT, NL, vol. 4, no. 1, 1 February 1994 (1994-02-01), pages 7-20, XP002112750, ISSN: 0923-179X

## Description

### FIELD OF THE INVENTION

The present invention relates to a method of purification of anti-CD20 antibodies comprising a cation exchange chromatography step.

### BACKGROUND OF THE INVENTION

Large-scale purification of proteins remains a significant challenge in the biopharmaceutical industry as efficient and cost-effective methods are required to achieve desired yields and purity levels. Therapeutic proteins are primarily products of recombinant DNA technology, i.e., cloning and expression of a heterologus gene in prokaryotic or eukaryotic systems. However, proteins expressed by recombinant DNA methods are typically associated with impurities such as host cell proteins (HCP), host cell DNA (HCD), viruses, etc. Also, there is significant heterogeneity in the expression of the desired protein, in the form of charged variants (typically acidic, lower pl variants and basic, higher pl variants). Further, multimeric proteins, such as antibodies, have a higher tendency to aggregate, contributing to significantly increased impurity levels.

The presence of these impurities, including aggregates and undesirable charged variants, is a potential health risk, and hence their removal from a final product is a regulatory requirement. Thus drug regulatory agencies such as United States Food and Drug administration (FDA) require that biopharmaceuticals be free from impurities, both product related (aggregates or degradation products) and process related (media components, HCP, DNA, chromatographic media used in purification, endotoxins, viruses, etc). See, *Office of Biologics Research and Review, Food and Drug Administration, Points to consider in the production and testing of new drugs and biologicals produced by recombinant DNA technology (Draft), 1985.* Thus, elimination of impurities from a final product is mandatory and poses a significant challenge in the development of methods for the purification of therapeutic proteins.

Antibodies constitute one of the most important classes of therapeutic proteins, especially in the areas of oncology, arthritis and other chronic diseases. The prior art discloses various methods for purification of crude or partially purified antibodies. WO 89/05157 teaches purification of immunoglobulins by cation-exchange chromatography using varying pH and salt concentrations in the wash and elution steps. WO 2004/024866 describes a method of purifying a polypeptide by ion exchange chromatography in which a gradient wash with differing salt concentrations is used to resolve the polypeptide. WO 1999/057134 describes the use of ion exchange chromatography for purification of polypeptides by varying conductivity and/or pH. US 5110913 claims purification of murine antibodies using low pH and at least three different pH conditions in the ion-exchange chromatographic step. WO2009058812 discloses the purification of anti-CD20 antibody rituximab by cation exchange chromatography. However the prior art typically describe the use of low pH wash conditions that may be accompanied by frequent and significant change in pH conditions during load/wash/elution steps. Such frequent alterations of pH conditions during chromatography may result in considerable reduction of antibody yield and, further, may decrease the stability of the antibody. Moreover frequent pH changes during chromatography pose difficulties in scale up.

Given the cumbersome nature of the conditions described in the prior art, alternative methods for purification of antibodies that alleviate some of the difficulties described above is desirable. The principle object of the present invention is to provide an improved method for obtaining antibody preparations that avoids use of low pH conditions and significantly reduces alterations in pH during a chromatographic step resulting in effective separation of charge variants, increased purity and recovery of the desired antibody.

### SUMMARY OF THE INVENTION

The present invention describes a process for the purification of anti-CD20 antibodies from a mixture of impurities using cation exchange chromatography wherein the pH of a first wash buffer is similar to the pH of the load buffer, and pH of a second wash buffer is similar to the pH of the elution buffer.

### BRIEF DESCRIPTION OF THE DRAWINGS

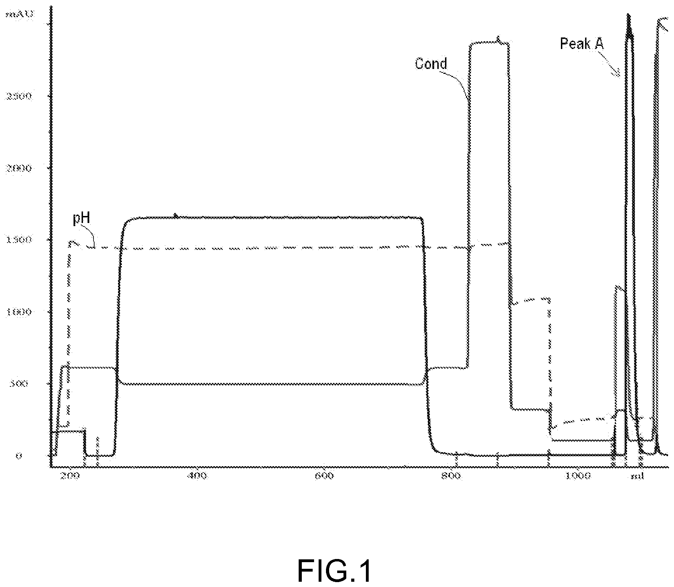

Fig. 1 is an illustration of a chromatogram from the procedure as described in Example 1. The line marked "Cond" represents the increase in conductivity in mS/cm. Peak A, represents the eluate obtained from protein A chromatography resin.

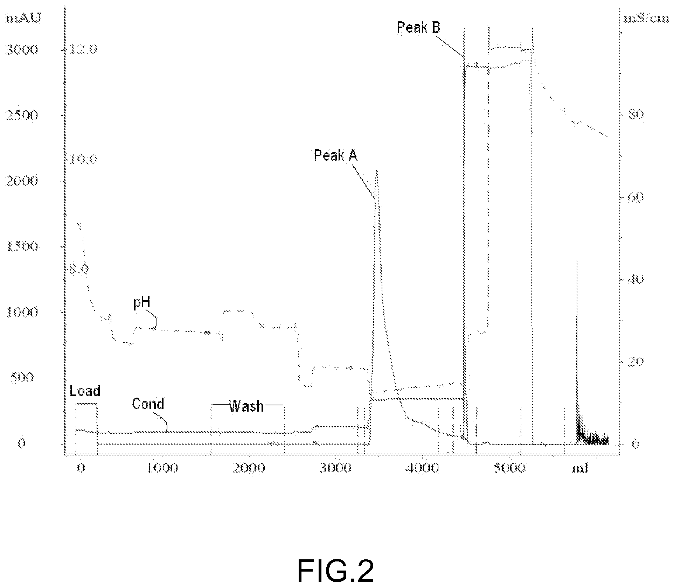

Fig. 2 is an illustration of a chromatogram from the procedure as described in Example 2. Peak A and B represent the eluate obtained from cation exchange chromatography. Peak A and B are charge variants of the anti-CD20 antibody.

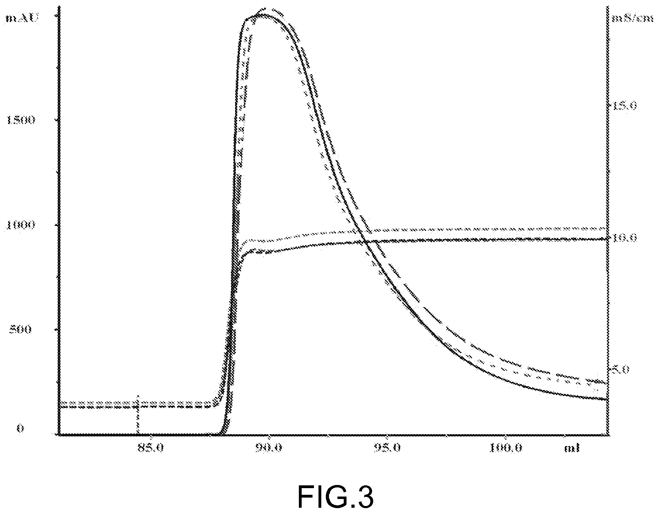

Fig. 3 is an illustration of a chromatogram from the procedure as described in Example 2. The consistency of elution in multiple runs is shown.

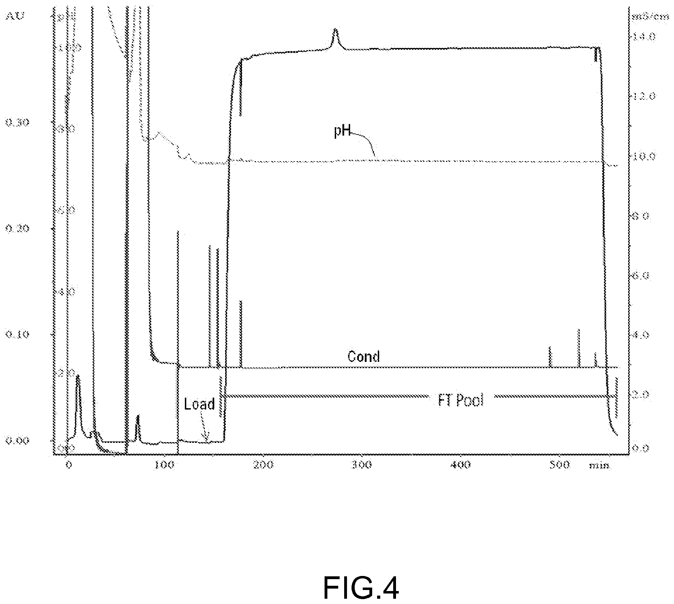

Fig. 4 is an illustration of a chromatogram from the procedure as described in Example 3. Figure represents the flow- through fraction of the anion exchange chromatography.

### DETAILED DESCRIPTION OF THE INVENTION

The present invention describes a process for purification of anti-CD20 antibodies by cation exchange chromatography. The chromatographic conditions require minimal pH adjustments during load/wash/elution steps. The process described in the present invention also avoids use of low pH buffers. The conditions described in the present invention results in effective separation of charged variants, as well as removal of impurities such as HCP, aggregates and Protein A leachates and an optimum yield of the desired antibody.

The term "antibody" as used herein refers to immunoglobulins and can be isolated from various sources, such as murine, human, recombinant etc. In its broadest sense it includes monoclonal antibodies, polyclonal antibodies, multispecific antibodies and antibody fragments. It also includes truncated antibodies, chimeric, humanized or pegylated antibodies, isotypes, allotypes and alleles of immunoglobulin genes and fusion proteins, which contain an immunoglobulin moiety.

The term "impurities" as used herein refers to a material that is different from the desired polypeptide. They may be nucleic acids such as host cell DNA, host cell proteins, variants of the desired polypeptide, another polypeptide, endotoxin etc.

The term "low pH" as used herein refers to a pH of less than 6.0

The term "load buffer" as used herein refers to the buffer that is used to load the composition comprising the antibody of interest and one or more impurities onto the ion exchange support.

The term "wash buffer" as used herein refers to a buffer that is used to wash or re-equilibrate the ion exchange support, or to elute one or more impurities from the ion exchange support, prior to elution of the antibody of interest.

The "elution buffer" is used to elute the antibody of interest from the ion exchange support.

In an embodiment, the invention provides a method of the purification of antibodies comprising,
a) Loading the antibody containing solution onto a cation exchange support using a buffer of pH value 7.5
b) Washing the support with a first wash buffer at a pH similar to the pH of the load buffer
c) Washing the support with a second wash buffer of pH value 6.5 and
d) Eluting the antibody from the support using an elution buffer at a pH similar to the second wash buffer.

In an embodiment, the pH of the second wash buffer is less than the pH of the first wash buffer.

In an embodiment, a protein-A chromatography, may precede the cation exchange chromatography.

In an embodiment, another ion-exchange chromatography or a hydrophobic interaction chromatography may precede or follow the cation exchange chromatography.

The embodiments mentioned herein may optionally include one or more tangential flow filtration, concentration, diafiltration or ultra filtration steps.

The embodiments mentioned herein optionally include one or more viral inactivation steps or sterile filtration or nano filtration steps.

The embodiments mentioned herein may include one or more neutralization steps.

The protein A chromatographic resin used may be any protein A or variant or a functional fragment thereof coupled to any chromatographic support. In embodiments, the protein A resin is Mabselect™ (GE-Healthcare Life sciences), an affinity matrix with recombinant Protein-A ligand. The resin is made of highly cross-linked agarose matrix.

Cation exchange chromatographic step mentioned in the embodiments may be carried out using any weak or strong cation exchange chromatographic resin or a membrane, which could function as a weak or a strong cation exchanger. Commercially available cation exchange support include a resin, but are not limited to, those having a sulfonate based group e.g., MonoS, MiniS, Source 15S and 30S, SP Sepharose Fast Flow, SP Sepharose High Performance from GE Healthcare, Toyopearl SP-650S and SP-650M from Tosoh, S-Ceramic Hyper D, from Pall Corporation or a carboxymethyl based group e.g., CM Sepharose Fast Flow from GE Healthcare, Macro-Prep CM from BioRad, CM-Ceramic Hyper D, from Pall Corporation, Toyopearl CM-650S, CM-650M and CM-650C from Tosoh. Alternatively, the support could be a monolithic column, disk or tubular, that performs the function of a cation exchanger. In embodiments of the invention, a strong cation exchange resin, such as SP-Sepharose® (GE Healthcare Life Sciences) is used. This resin is made using a highly cross-linked, 6 % agarose matrix attached to a sulfopropyl functional group.

Anion exchange chromatography mentioned in the embodiments may be carried out using any weak or strong anion exchange chromatographic resin or a membrane which could function as a weak or a strong anion exchanger. Commercially available anion exchange resins include, but are not limited to, DEAE cellulose, Poros PI 20, PI 50, HQ 10, HQ 20, HQ 50, D 50 from Applied Biosystems, MonoQ, MiniQ, Source 15Q and 3OQ, Q, DEAE and ANX Sepharose Fast Flow, Q Sepharose high Performance, QAE SEPHADEX and FAST Q SEPHAROSE from GE Healthcare, Macro-Prep DEAE and Macro-Prep High Q from Biorad, Q-Ceramic Hyper D, DEAE-Ceramic Hyper D, from Pall Corporation. In embodiments of the invention, a strong anion exchange resin, such as Q- Sepharose Fast Flow® (GE Healthcare Life Sciences) is used. This resin is made of highly cross-linked, 6 % agarose matrix attached to -O-CH₂CHOHCH₂OCH₂CHOHCH₂N⁺(CH₃)₃ functional group.

Examples of buffering agents used in the buffer solutions include, but are not limited to, TRIS, phosphate, citrate, acetate, succinate, MES, MOPS, or ammonium and their salts or derivatives thereof.
The invention is more fully understood by reference to the following examples.

### EXAMPLE 1

### Protein A chromatography

An anti-CD20 antibody was cloned and expressed in a CHO cell line as described in U.S. Patent No. 7,381,560. The cell culture broth containing the expressed antibody was harvested, clarified and subjected to protein A affinity chromatography as described below.
The clarified cell culture broth was loaded onto a protein A chromatography column (Mabselect, VL44x250, 205 mL) that was pre-equilibrated with Tris buffer solution (pH 7.0). The column was then washed with equilibration buffer. This was followed by a wash with Tris buffer (pH 7.0) with higher conductivity and a final wash with citrate buffer at pH 5. The bound antibody was eluted using citrate buffer, pH 2.5 - 3.5.

### EXAMPLE 2

### Cation exchange chromatography

The eluate obtained from the protein A chromatography procedure described in Example 1 was loaded onto a cation exchange resin (SP Sepharose, VL44x250, 304 mL) pre-equilibrated with Tris buffer (pH 7.5) at a conductivity of 3.0 to 6.0 mS/cm. This was followed by washing the resin with a wash buffer of Tris buffer (pH 7.5) at a conductivity of 3.0 to 6.0 mS/cm. A second wash step was performed with wash buffer consisting of citrate buffer, pH 6.5, at a conductivity of 3.0 to 6.0. The bound antibody was eluted using a buffer of citrate buffer, pH 6.5 at conductivity between 9-12 mS/cm.

### EXAMPLE 3

### Anion exchange chromatography

The eluate obtained from the cation exchange chromatography procedure described in Example 2 was loaded onto an anion exchange resin (Q-Sepharose FF, VL32x250, 80 mL) pre-equilibrated with a Tris buffer (pH 7.5) equilibration buffer. This was followed by a post load wash with equilibration buffer and the load and wash flow-through was collected.

**Table 1:**

| **Sample** | **Protein-A leachate (ng/mg)** | **Host Cell Protein (ng/mg)** | **Aggregate (%)** | **Step Recovery (%)** |
|---|---|---|---|---|
| Clarified cell culture broth | - | 2202 | -- | 98 |
| Protein A eluate | - | 4.0 | 6.33 | 86.4 |
| Cation Exchange chromatography eluate | 0.17 | 0.3 | 0.81 | 81.1 |
| Anion Exchange chromatography flow through | 0.08 | BDL | 0.67 | 95.6 |

| | | | | |
|---|---|---|---|---|
| BDL: Below Detection Limit | | | | |

**Table 2:**

| **Sample** | **Acidic variants (%)** | **K0 (%)** | **Basic Variant-1 (%)** | **Basic Variant-2 (%)** |
|---|---|---|---|---|
| Protein A eluate | 10.64 | 18.03 | 64.09 | 7.24 |
| Cation Exchange chromatography eluate | 13.73 | 23.30 | 62.97 | BDL |
| Anion Exchange chromatography flow through | 14.03 | 23.60 | 62.37 | BDL |

| | | | | |
|---|---|---|---|---|
| K0: Species devoid of the C-terminal lysine residue BDL: Below Detection Limit | | | | |

## Claims

1. A process for purification of an anti-CD20 antibody from a solution containing the antibody and impurities, comprising,
a) loading the antibody containing solution onto a cation exchange chromatography support using a buffer of pH value 7.5,
b) washing the support with a first wash buffer at a pH similar to the pH of the load buffer,
c) washing the support with a second wash buffer of pH value 6.5 and
d) eluting the antibody from the support with an elution buffer at a pH similar to the second wash buffer.

2. A process according to claim 1, wherein the pH value of the first wash buffer is 7.5.

3. A process according to claim 1, wherein the pH value of the elution buffer is about 6.5.

4. A process according to claim 1, wherein the cation-exchange chromatography is preceded by a protein-A affinity chromatography step.

5. A process according to claim 1, wherein the cation-exchange chromatography is preceded by an anion exchange chromatography step or a hydrophobic interaction chromatography step.

6. A process according to claim 1, wherein the cation- exchange chromatography step is followed by an anion exchange chromatography step or a hydrophobic interaction chromatography step.

7. A process according to claim 1, wherein the cation- exchange chromatography step is preceded and/or followed by one or more ion exchange chromatography steps.

## Patentansprüche

1. Verfahren zum Reinigen eines Anti-CD20-Antikörpers von einer Lösung, die die Antikörper und Verunreinigungen enthält, das Folgendes beinhaltet:
a) Laden der Antikörper-haltigen Lösung auf einen Kationenaustauschchromatographieträger mit einem Puffer mit einem pH-Wert von 7,5,
b) Waschen des Trägers mit einem ersten Waschpuffer bei einem pH-Wert ähnlich dem pH-Wert des Ladepuffers,
c) Waschen des Trägers mit einem zweiten Waschpuffer mit einem pH-Wert von 6,5, und
d) Eluieren des Antikörpers von dem Träger mit einem Elutionspuffer bei einem pH-Wert ähnlich dem des zweiten Waschpuffers.

2. Verfahren nach Anspruch 1, wobei der pH-Wert des ersten Waschpuffers 7,5 ist.

3. Verfahren nach Anspruch 1, wobei der pH-Wert des Elutionspuffers etwa 6,5 ist.

4. Verfahren nach Anspruch 1, wobei der Kationenaustauschchromatographie ein Protein-A-Affinitätschromatographieschritt vorangeht.

5. Verfahren nach Anspruch 1, wobei der Kationenaustauschchromatographie ein Anionenaustauschchromatographieschritt oder ein Hydrophobe-Interaktion-Chromatographieschritt vorangeht.

6. Verfahren nach Anspruch 1, wobei auf den Kationenaustauschchromatographieschritt ein Anionenaustauschchromatographieschritt oder ein Hydrophobe-Interaktion-Chromatographieschritt folgt.

7. Verfahren nach Anspruch 1, wobei dem Kationenaustauschchromatographieschritt ein oder mehrere Ionenaustauschchromatographieschritte vorangehen und/oder folgen.

## Revendications

1. Procédé de purification d'un anticorps anti-CD20 d'une solution contenant l'anticorps et des impuretés, comprenant :
a) charger la solution contenant l'anticorps dans un support de chromatographie par échange de cations en utilisant un tampon d'une valeur de pH de 7,5,
b) laver le support avec un premier tampon de lavage à un pH semblable au pH du tampon de charge,
c) laver le support avec un deuxième tampon de lavage d'une valeur de pH de 6,5 et
d) éluer l'anticorps du support avec un tampon d'élution à un pH semblable au deuxième tampon de lavage.

2. Procédé selon la revendication 1, dans lequel la valeur de pH du premier tampon de lavage est de 7,5.

3. Procédé selon la revendication 1, dans lequel la valeur de pH du tampon d'élution est d'environ 6,5.

4. Procédé selon la revendication 1, dans lequel la chromatographie par échange de cations est précédée par une étape de chromatographie d'affinité par protéine A.

5. Procédé selon la revendication 1, dans lequel la chromatographie par échange de cations est précédée par une étape de chromatographie par échange d'anions ou par une étape de chromatographie par interaction hydrophobe.

6. Procédé selon la revendication 1, dans lequel l'étape de chromatographie par échange de cations est suivie par une étape de chromatographie par échange d'anions ou par une étape de chromatographie par interaction hydrophobe.

7. Procédé selon la revendication 1, dans lequel l'étape de chromatographie par échange de cations est précédée et/ou suivie par une ou plusieurs étapes de chromatographie par échange d'ions.
